**Europäisches Patentamt**

**European Patent Office**

**Office européen des brevets**

(19)

(11)  **EP 0 809 484 B1**

(12)  **FASCICULE DE BREVET EUROPEEN**

(45) Date de publication et mention
de la délivrance du brevet:
**29.11.2000  Bulletin 2000/48**

(21) Numéro de dépôt: **96904153.2**

(22) Date de dépôt: **16.02.1996**

(51) Int Cl.$^7$: **A61K 7/48**, A61K 35/78

(86) Numéro de dépôt international:
**PCT/FR96/00256**

(87) Numéro de publication internationale:
**WO 96/25143 (22.08.1996 Gazette 1996/38)**

(54) **COMPOSITION COSMETIQUE OU PHARMACEUTIQUE, NOTAMMENT DERMATOLOGIQUE, CONTENANT UN EXTRAIT DE BERTHOLLETIA**

BERTHOLLETIA ENTHALTENDE KOSMETISCHE ODER PHARMAZEUTISCHE, INSBESONDERE DERMATOLOGISCHE ZUSAMMENSETZUNG

COSMETIC OR PHARMACEUTICAL, PARTICULARLY DERMATOLOGICAL, COMPOSITION CONTAINING A BERTHOLLETIA EXTRACT

(84) Etats contractants désignés:
**CH DE FR GB IT LI**

(30) Priorité: **17.02.1995  FR 9501840**

(43) Date de publication de la demande:
**03.12.1997  Bulletin 1997/49**

(73) Titulaire: **LVMH RECHERCHE
75008 Paris (FR)**

(72) Inventeurs:
  • **BONTE, Frédéric
    F-45100 Orléans (FR)**
  • **DUMAS, Marc
    F-45100 Orléans (FR)**
  • **LAVAUD, Catherine
    F-51430 Tinqueux (FR)**
  • **MASSIOT, Georges
    F-51100 Reims (FR)**

(74) Mandataire: **Giraud, Françoise
    Cabinet Beau de Loménie
    158, rue de l'Université
    75340 Paris Cedex 07 (FR)**

(56) Documents cités:
  • **DATABASE CHEMABS CHEMICAL ABSTRACTS SERVICE, COLUMBUS, OHIO, US 106:118408, S.S. SUN ET AL: "Brazil nut (Bertholletia excelsa HBK) proteins: fractionation, composition, and identification of a sulfur-rich protein" XP002004184**
  • **DATABASE CHEMABS CHEMICAL ABSTRACTS SERVICE, COLUMBUS, OHIO, US DN: 75:34197, F. TATEO: "Acid composition of the fatty material extracted from the seeds of Bertholletia excelsa" XP002004185**

EP 0 809 484 B1

**Description**

La présente invention concerne essentiellement une composition cosmétique ou pharmaceutique, notamment dermatologique, contenant un extrait de Bertholletia.

Plus précisément, la présente invention concerne l'utilisation d'un extrait de Bertholletia comme agent cosmétique, ou pour la préparation d'une composition cosmétique ou pharmaceutique, notamment dermatologique, stimulant notamment la synthèse du collagène ou ayant une activité antiradicalaire, et destinée en particulier à lutter contre les effets du vieillissement cutané, à obtenir un raffermissement cutané, à améliorer la cicatrisation ou à traiter les diverses pathologies accompagnées d'une déficience en collagène, ainsi que des compositions cosmétiques ou pharmaceutiques en comportant application.

La plante Bertholletia appartient à la famille des Lecythidacées. Il s'agit d'un genre qui comprend deux espèces extrêmement voisines : Bertholletia excelsa et Bertholletia nobilis. Ce sont des arbres des régions tropicales de l'Amérique du Sud, que l'on rencontre en particulier au Brésil. Ils peuvent atteindre 30 à 40 mètres de hauteur. On les appelle communément noyers ou châtaigniers du Brésil. Les noix des deux espèces sont à peu près identiques et sont pratiquement confondues. Elles sont utilisées pour leurs qualités alimentaires.

On en trouve une description notamment dans l'ouvrage "Dictionnaire des Huiles Végétales" par Paul H. Mensier, Editions Paul Lechevalier, Paris, 1957.

En Amazonie, l'écorce du tronc de Bertholletia excelsa, qui contient notamment des saponines, est utilisée en infusion contre les maladies du foie. L'huile de ses graines peut servir à la fabrication de savons (R.E. Schultes et al. The healing forest. Discorides Press, Portland, Oregon, USA, 1990, vol. 2, pages 225-226).

Il a maintenant été découvert que des extraits de Bertholletia présentaient un grand intérêt en cosmétique, notamment pour le soin de la peau.

En particulier, il a été découvert une activité surprenante des extraits de Bertholletia sur la synthèse de collagène, en particulier de collagène de type I ou de type IV, ci-après dénommé en abréviation "collagène I" ou "collagène IV".

On sait que la peau contient essentiellement du collagène I, protéine synthétisée par les fibroblastes, cellules majoritaires du derme. Cette protéine joue un rôle de soutien et est responsable des qualités rhéologiques du derme, en particulier de sa fermeté et du maintien de son architecture (E.U. KUCHARZ, "The collagens : Biochemistry and pathophysiology", Springer Verlag, Berlin 1992). Par ailleurs, il a été montré que les fibroblastes du derme des personnes âgées sécrètent moins de collagène que ceux des sujets jeunes (M. DUMAS et al, Mech, Ageing Dev. (1994) 73, 179-187). Ainsi, avec l'âge, se produit une diminution des qualités rhéologiques de la peau et de sa réponse aux contraintes auxquelles elle est soumise quotidiennement. La peau se distend, réagit moins bien aux tensions, perd son tonus et les rides se creusent.

On sait aussi que le collagène IV est un collagène majeur de la jonction dermo-épidermique. On pourra se rapporter à ce sujet à l'article de Briggaman R.A., intitulé "Biochemical composition of the epidermal-dermal junction and other basement membranes", publié dans J. Invest. Dermatol. 78 (1982), pages 1-6, ou l'article de P. Verando intitulé "La jonction dermoépidermique" publiée dans le Séminaire Inserm, vol. 214, (1991), pages 83-100, édition Inserm.

Ce collagène IV qui se trouve au niveau de la lamina densa, intervient dans l'attachement cellulaire et est synthétisé par les kératinocytes. Les plaques d'ancrages assurent les liaisons entre les fibriles d'ancrages (collagène VII) liées aux kératinocytes et les fibres du collagène du derme (collagène I et III). Ces plaques d'ancrage sont constituées de collagène IV. Elles sont essentielles dans le maintien d'une interface fonctionnelle entre les deux compartiments cutanés. Le collagène IV joue également un rôle important dans les processus de cicatrisation. Il permet la formation d'une peau cicatricielle de bonne qualité.

Il a, d'autre part, été découvert que des extraits de Bertholletia présentaient une activité antiradicalaire remarquable, c'est-à-dire qu'ils présentent une fonction de piégeurs de radicaux libres. Les effets bénéfiques pour la peau de piégeurs de radicaux libres ont été récemment rappelés par F.J. Wright dans J. Appl. Cosmetol. 13 (1995), pages 41-50.

Or, il est reconnu que les radicaux libres interviennent dans les phénomènes de vieillissement en particulier au niveau cutané et modifient les qualités et les propriétés de la peau. On pourra se reporter à ce sujet à l'ouvrage de 1. Emerit ayant pour titre "Free radicals and aging", I. Emerit, B. Chance EDS, Birkhauser Verlag, Bâle, 1992, pages 328-341.

De ce fait, l'action des extraits de Bertholletia sur la synthèse de collagène ou sur les radicaux libres rend ceux-ci particulièrement utiles pour lutter contre les effets de vieillissement cutané, tels que les rides ou le relâchement des tissus du support cutané, ou pour améliorer la cicatrisation.

La présente invention a donc pour but principal de résoudre le nouveau problème technique consistant en la fourniture d'une nouvelle formulation d'une compositior cosmétique ou pharmaceutique, notamment dermatologique, présentant une bonne efficacité sur la prévention ou le traitement des effets du vieillissement cutané, ainsi que sur le raffermissement cutané, ou pour améliorer la cicatrisation, ou présentant une bonne efficacité antiradicalaire.

La présente invention a encore pour but principal de résoudre ce nouveau problème technique d'une manière particulièrement simple, satisfaisante et utilisable à l'échelle industrielle, notamment dans l'industrie cosmétique ou pharmaceutique.

Ainsi, selon un premier aspect, la présente invention concerne l'utilisation d'un extrait de Bertholletia comme agent cosmétique, notamment à activité antiradicalaire, en particulier pour le soin de la peau.

Selon une variante de réalisation de l'invention, on utilise un extrait de Bertholletia, en particulier de Bertholletia excelsa, pour la préparation d'une composition cosmétique ou pharmaceutique, notamment dermatologique, stimulant notamment la synthèse du collagène, en particulier celle du collagène I ou du collagène IV, ou à activité antiradicalaire, et destinée notamment à améliorer la cicatrisation cutanée ainsi que les propriétés biomécaniques et l'aspect de surface de la peau, à obtenir un raffermissement cutané, à lutter contre les effets du vieillissement cutané, ou à traiter les diverses pathologies accompagnées d'une déficience en collagène.

Suivant une variante de réalisation avantageuse, l'extrait de Bertholletia est utilisé pour la préparation d'une composition à effet anti-vieillissement cutané, en particulier anti-rides, destinée à prévenir l'apparition des rides ou en atténuer la profondeur.

Selon une autre variante, l'extrait de Bertholletia est un extrait d'écorce de cette plante, en particulier un extrait de tronc de la plante Bertholletia excelsa.

Selon une autre variante de réalisation, l'extrait de Bertholletia est un extrait des fruits, c'est-à-dire des noix, de Bertholletia, de préférence des extraits de péricarpe des noix de Bertholletia, et encore mieux de Bertholletia excelsa.

En particulier, l'extrait de Bertholletia peut être obtenu selon le procédé décrit ci-après à titre indicatif, mais nullement limitatif.

On effectue une première extraction de l'écorce ou des noix, en particulier l'écorce du tronc ou le péricarpe de noix, de la plante par un solvant polaire, avantageusement choisi parmi le groupe constitué par : l'eau, les alcools comportant de préférence de 1 à 4 atomes de carbone, les solvants chlorés comportant de préférence 1 à 2 atomes de carbone, les esters organiques comportant de préférence de 3 à 6 atomes de carbone ; et d'un solvant mixte à base d'un mélange quelconque des solvants précités.

Encore de préférence, le solvant de première extraction est choisi parmi le groupe consistant de l'eau, le méthanol, l'éthanol, un mélange méthanol-eau ou un mélange éthanol-eau, le chloroforme et le dichlorométhane. Encore de préférence, il s'agit de l'eau, du méthanol, de l'éthanol ou d'un mélange quelconque de ces solvants.

Le rapport de l'écorce ou des noix, en particulier le péricarpe de noix, à l'agent d'extraction n'est pas critique et généralement sera compris entre 1 : 5 et 1 : 20 parties en poids.

L'extraction s'effectue généralement à des températures comprises entre la température ambiante et le point d'ébullition du solvant utilisé pour l'extraction.

De préférence, cette première extraction est effectuée au reflux sous pression atmosphérique pendant une durée de 2 à 4 h. En outre, elle est avantageusement précédée d'une macération à froid pendant 2 à 4 h dans le solvant d'extraction.

En fin d'extraction, la phase du solvant contenant l'extrait est filtrée puis concentrée et/ou évaporée à sec sous pression réduite. On obtient ainsi un premier extrait brut de Bertholletia selon l'invention. Cet extrait brut peut être purifié selon divers procédés bien connus à l'homme de l'art.

En particulier, si l'on souhaite enrichir en saponines l'extrait de Bertholletia selon l'invention, on opère selon le procédé décrit ci-après à titre indicatif, mais nullement limitatif. L'extrait obtenu après la première extraction est évaporé à sec. Le résidu est ensuite repris par du méthanol. La solution obtenue est versée dans de l'acétone. Il se forme alors un précipité que l'on récupère par filtration. De préférence, ce précipité est séché, puis dialysé contre de l'eau déminéralisée. Enfin, on lyophilise pour obtenir un extrait sec de Bertholletia enrichi en saponines, selon l'invention.

Les procédés d'extraction décrits ci-dessus s'appliquent en particulier à l'espèce Bertholletia excelsa.

Selon un second aspect, la présente invention concerne aussi une composition cosmétique caractérisée en ce qu'elle comprend, à titre d'ingrédient actif, une quantité cosmétiquement efficace d'un extrait de Bertholletia, en particulier de Bertholletia excelsa, de préférence dispersé dans un excipient cosmétiquement acceptable.

Selon une variante de réalisation particulière, cette composition cosmétique stimulant la synthèse du collagène, en particulier celle du collagène I ou du collagène IV, ou ayant une activité antiradicalaire, est destinée notamment à lutter contre les effets du vieillissement cutané, ou à obtenir un raffermissement cutané. Une telle composition, par exemple, peut avantageusement être utilisée comme composition pour prévenir l'apparition des rides ou en atténuer la profondeur.

Selon un troisième aspect, l'invention concerne encore une composition pharmaceutique, notamment dermatologique, stimulant la synthèse du collagène, en particulier celle du collagène I ou du collagène IV, ou à activité antiradicalaire, caractérisée en ce qu'elle comprend, à titre d'ingrédient actif, une quantité pharmaceutiquement efficace d'un extrait de Bertholletia, en particulier de Bertholletia excelsa, dispersé dans un excipient pharmaceutiquement acceptable.

Selon un mode de réalisation particulier, ladite composition est destinée à améliorer la cicatrisation cutanée ou à traiter les diverses pathologies accompagnées d'une déficience en collagène, ou résultant de la présence de radicaux libres.

Selon un quatrième aspect, l'invention couvre encore un procédé de traitement cosmétique ou pharmaceutique, notamment dermatologique, caractérisé en ce qu'il comprend l'application d'une quantité cosmétiquement ou pharmaceutiquement efficace pour un traitement cosmétique ou pharmaceutique d'un extrait de Bertholletia, en particulier Bertholletia excelsa, en particulier dispersé dans un excipient cosmétiquement ou pharmaceutiquement acceptable. De préférence, il s'agit d'une application topique cutanée sur des zones de la peau concernée. Les applications cosmétiques ou pharmaceutiques particulièrement avantageuses actuellement résultent de la description précédente ainsi que de la description suivante en relation avec les exemples, et des revendications. Il en est de même en ce qui concerne la concentration en extrait.

Dans l'un ou l'autre des aspects précédents, on utilisera de préférence l'extrait de Bertholletia, en particulier Bertholletia excelsa, à une concentration comprise entre 0,0001 % et 1 % en poids par rapport au poids total de la composition finale. De préférence, cette concentration est comprise entre 0,01 % et 0,25 % en poids par rapport au poids total de la composition finale.

Egalement dans l'un quelconque des aspects précédents, la composition selon l'invention contient de préférence en outre une substance active choisie parmi le groupe constitué par l'acide ascorbique, l'acide madécassique, l'acide asiatique, le madécassoside, l'asiaticoside, l'alpha-1-protéinase inhibiteur, les inhibiteurs de collagénase, tels que l'acide rétinoïque, les inhibiteurs d'élastase, la lysine, la proline, le 2-oxoglutarate, et le ginsénoside $R_0$.

D'autres buts, caractéristiques et avantages de l'invention apparaîtront clairement à la lecture de la description explicative qui va suivre, faite en référence à plusieurs exemples donnés uniquement à titre d'illustration et qui ne sauraient par conséquent en aucune façon limiter la portée de l'invention.

Dans les exemples, les pourcentages sont exprimés en poids, sauf indication contraire. Dans le cas des extraits, les quantités de ceux-ci sont exprimées en poids sec.

Exemple 1

Préparation d'un extrait méthanolique d'écorce du tronc de Bertholletia excelsa, enrichi en saponines

On prépare de la poudre d'écorce de tronc de Bertholletia excelsa par broyage. On fait ensuite macérer 49 g de cette poudre pendant 2 h dans 500 ml de méthanol. On porte le tout à reflux pendant 3 h, puis on laisse refroidir et on filtre sur verre fritté. Ce filtrat obtenu constitue un premier extrait selon l'invention, dit extrait $I_1$. Ce premier extrait est évaporé à sec. Le résidu, pesant 14,7 g, est repris par 100 ml de méthanol. On effectue une précipitation en versant cette solution dans 500 ml d'acétone, puis on filtre. Le précipité est ensuite séché sur un agent déshydratant solide tel que de la potasse, on obtient ainsi 1,33 g de produit sec. Une quantité de 830 mg de ce produit est ensuite dialysée pendant 4 jours contre 9 ml d'eau déminéralisée, puis on lyophilise. On obtient ainsi 182 mg d'extrait enrichi en saponines selon l'invention, dit extrait $I_2$.

Exemple 2

Préparation d'un extrait méthanolique de péricarpe de noix de Bertholletia excelsa du brésil, enrichi en saponines

On rassemble les péricarpes de noix de Bertholletia excelsa du brésil disponibles dans le commerce que l'on broie grossièrement et finement.

On prend alors 100 g de la poudre de péricarpe de noix ainsi obtenue et on l'extrait successivement trois fois par 1 l de méthanol au reflux pendant 30 min.

Chaque extrait est filtré sur un filtre de diamètre de pore de 0,45 μm et les troix extraits sont réunis.

On concentre à l'aide d'un évaporateur rotatif jusqu'à obtention d'un film sec.

Ce film est repris sous agitation par de l'eau. Le lait ainsi obtenu est lyophilisé.

On obtient ainsi environ 5 g d'extrait de péricarpe de noix enrichi en saponines selon l'invention, dit extrait $I_3$. On notera que le rendement de l'extraction est voisin de 5 %.

Exemple 3

Mise en évidence de l'activité d'un extrait méthanolique d'écorce de Bertholletia excelsa préparé selon l'exemple 1, extrait $I_2$, sur la synthèse de collagène par des fibroblastes humains en culture

Culture de fibroblastes

Des cultures de fibroblastes de derme adulte sain sont réalisées en utilisant la méthode par explants à l'aide d'un prélèvement de peau faciale obtenu sur une femme âgée de 60 ans au cours d'un lifting.

Les fibroblastes sont cultivés jusqu'à confluence dans un milieu E 199 (Gibco) supplémenté avec 2 mM de L-glutamine (Gibco) et 10 % en v/v de sérum de veau foetal (Gibco) à 37°C dans une atmosphère à 5 % de $CO_2$ humidifiée. Pour évaluer la teneur en collagène, les cultures primaires en confluence sont récoltées avec une solution à 0,1 % de trypsine et 0,02 % d'EDTA dans une solution saline tamponnée de phosphate (PBS) à pH 7,2 et les cellules sont alors ensemencées à la densité de $10^4$ fibroblastes par puits, dans des plaques de microculture (Falcon) à 96 puits, en présence du même milieu de culture que celui décrit ci-dessus.

24 h après l'ensemencement, le milieu est enlevé et remplacé par un milieu de même composition que le milieu décrit précédemment, si ce n'est qu'il ne contient pas de sérum et que l'on a ajouté 25 µM d'acide L-ascorbique sous forme de sel de sodium. En outre, ce nouveau milieu contient ou non le produit à tester (extrait $I_2$) selon qu'il s'agit d'une culture traitée ou d'une culture témoin. On laisse ensuite incuber pendant une période supplémentaire de 48 h à 37°C. Le produit à tester (extrait $I_2$) a été dissous dans le DMSO avant l'incorporation dans le milieu de culture (la concentration finale en DMSO dans le milieu est de 0,1 % en v/v).

Viabilité des cellules

A la fin de la période d'incubation, le milieu est enlevé et un test de viabilité de cellules MTT a été réalisé conformément à la publication de Denizot F. et al. J. Immunol. Mcthods, (1986) 89, 271-277. Les cellules sont incubées avec 101 µl d'une solution en sel tétrazolium à 0,5 mg/ml (bromure de 3-[4,5-diméthylthiazol-2-yl]-2,5-diphényltétrazolium ou MTT) dans le milieu E 199 sans rouge de phénol (Gibco) pendant 3 h à 37°C. Ensuite, 100 µl d'isopropanol sont ajoutés à chaque puits pour solubiliser le dérivé bleu de Formazan sombre formé par les cellules vivantes. On mesure le taux d'absorbance à 540 nm.

Mesure du collagène

La quantité totale de collagène de type I sécrété par les fibroblastes, qui se trouve soit associé aux cellules, soit libéré dans le milieu dépourvu de sérum après 48 h d'incubation avec ou sans le produit (extrait $I_2$), est déterminée par un test ELISA comme précédemment décrit dans la publication de Dumas M. et al., Mech. Agcing Dev. (1994) 73, 179-187, et celle de Grimaud J.P. et al., dans : Methods of Enzymatic Analysis (Bermeyer, H.U., ed.), VCH Publishers, Weinheim, (1986), 186-201.

Les milieux d'incubation dépourvus de sérum et les cellules restantes homogénéisées par sonication dans de la glace sont recueillis et transférés dans des puits d'une immunoplaque en plastique (NUNC) pour une incubation de 24 h à +4°C pour permettre l'adhérence du collagène sécrété. Des inhibiteurs de protéase (éthylmaléimide, fluorure de phénylméthylsulfonyle, éthylènediaminetétraacétate, chacun en concentration finale de 1 mM) sont ajoutés pendant cette période. Les plaques sont ensuite rincées avec le PBS. Une étape de lavage similaire est réalisée après chaque traitement de plaque.

Après 24 h d'incubation à 4°C avec de la sérum albumine pour éviter une liaison non spécifique, des anticorps de lapin anti-collagène de type I humain (Institut Pasteur, Lyon, France) sont ajoutés pendant 1,5 h à 22°C et les anticorps liés sont mis à réagir avec de l'IgG de chèvre anti-lapin conjugué à de la phosphatase alcaline (Interchim, Montluçon, France). L'absorbance du para-nitrophénol formé à partir de para-nitrophénylphosphate (Sigma) par la phosphatase alcaline est mesurée à 405 nm. Les densités optiques sont converties en nanogramme de collagène en utilisant une courbe étalon établie avec du collagène de type I humain purifié (Institut Jacques Boy, France).

Statistiques

Les taux de collagène (moyenne ± SD, déviation standard, n = 6) sont comparés à ceux déterminés à partir des cultures témoins non traitées. La significativité de cette comparaison est appréciée pour des valeurs de p inférieures à 0,05 par le test t de Student.

Résultats

On calcule le pourcentage de stimulation A de la synthèse de collagène I en comparant les quantités de collagène I sécrétées dans les cultures témoins (ne recevant aucun produit à tester) $C_o$ et dans les cultures traitées $C_t$, à partir de la formule suivante :

$$A = \frac{C_t - C_o}{C_o} \times 100$$

L'ensemble des résultats figure au tableau I ci-dessous.

TABLEAU I

| Cultures | % de viabilité/témoin | Collagène I sécrété par fibroblastes (ng/10000 cellules/48 h) | % de stimulation A | Significativité |
|---|---|---|---|---|
| Culture témoin sans produit | 100 | 924 | 0 | |
| Produit de l'invention $I_2$ à 1 µg/ml | 100 | 1359 | + 47 | S* |
| Produit de l'invention $I_2$ à 2,5 µg/ml | 100 | 2163 | + 134 | S* |

*S = significatif

L'examen du tableau I montre d'abord que le taux de viabilité des cellules dans les cultures traitées n'a pas varié de manière significative par rapport à celui des cultures témoins. L'extrait $I_2$ ne présente donc pas de cytotoxicité.
Dans ce tableau, on a exprimé les quantités de collagène I sécrétées par les fibroblastes en ng/10000 cellules/ 48 h.
On observera, à partir du tableau I, que l'extrait méthanolique de Bertholletia excelsa a produit une activité significative de stimulation de la synthèse de collagène I par les fibroblastes.
Ainsi, l'extrait de Bertholletia excelsa peut être utilisé pour des produits destinés à combattre le vieillissement cutané ou nécessitant une augmentation de synthèse locale de collagène comme cela est le cas lors d'un traitement des rides ou pour contribuer à l'amélioration de la cicatrisation cutanée.

Exemple 4

Mise en évidence de l'activité d'un extrait méthanolique de péricarpe de noix de Bertholletia excelsa selon l'invention, l'extrait $I_3$, sur la synthèse de collagène IV par des kératinocytes humains en culture

Culture des kératinocytes

Des kératinocytes humains provenant d'un lifting d'une femme de 60 ans, peau caucasienne, sont cultivés dans un milieu de croissance Gibco spécifique des kératinocytes SFM-K complémenté en EGF (Epidermal Growth Factor) et extrait pituitaire (Gibco, France). Les cellules sont ensemencées à raison de 30 000 par puits de culture dans ce milieu SFM-K.
Après 24 h, le milieu d'ensemencement est retiré et remplacé par du milieu SFM-K dilué au 1/50 dans le même milieu de base mais dénué de EGF et d'extrait pituitaire. On ajoutera alors le produit à tester (dissout dans le DMSO) aux différentes concentrations pendant 48 h. Les cultures témoins recevront la même quantité de solvant du produit testé, ici le DMSO (0,1 % final).
Les surnageants de culture sont récupérés et le collagène IV dosé selon une technique classique ELISA précédemment décrite dans Dumas M. et al., Mechanisms of Ageing and Development, 73, 1994, pages 179-187. L'anticorps anti-collagène IV humain utilisé est fourni par l'Institut Pasteur, Lyon, France.
Parallèlement, un dosage global des protéines est effectué par la technique à l'acide bicinchoninique, kit BCA commercialisé par Sigma, France.
Les résultats obtenus sont rapportés au tableau II ci-après :

TABLEAU II

| PRODUIT | Collagène IV ng/100 µg de protéines | Pourcentage d'activité | Significativité |
|---|---|---|---|
| Témoin DMSO | 1,499 ± 0,635 | | |
| Produit de l'invention $I_3$ à 1 µg/ml | 2,983 ± 0,613 | + 99 | S* |
| Produit de l'invention $I_3$ à 10 µg/ml | 3,153 ± 0,732 | + 110 | S* |

*S = significatif au seuil de 5 %

L'examen du tableau II montre très clairement que l'extrait testé stimule significativement la synthèse de collagène IV par les kératinocytes humains en culture. Une de ses applications est donc le renforcement de la structure et des propriétés de la jonction dermo-épidermique, zone d'échange entre le derme et l'épiderme et zone très importante pour les processus de différenciation des kératinocytes.

Exemple 5

Mise en évidence de l'activité antiradicalaire d'un extrait méthanolique de péricarpe de noix de Bertholletia excelsa

Dans cet essai, on utilise l'extrait $I_3$ de l'exemple 2. Ce test est basé sur la mesure de viabilité cellulaire de kératinocytes humains normaux soumis à une agression radicalaire par un système HX/XO hypoxanthine/xanthine oxydase.

Ce gain de viabilité cellulaire avec et sans produit dans les cultures sera évalué selon une méthode comparable à celle décrite par M.S. Noël - Hudson et al. dans International Journal of Cosmetic Science 1990, 12, pages 105-114, qui est incorporé ici dans sa totalité par référence.

Protocole du test

Des kératinocytes de prépuce sont cultivés dans un milieu K-SFM de chez Gibco, France, complémenté avec de l'EGF et des extraits de glande pituitaire bovine. Le milieu est changé tous les deux jours jusqu'à confluence.

Après trypsinisation, les cellules sont mises dans des boites 96 puits à la concentration de 20 000 cellules par puits. On les laisse alors proliférer dans du milieu K-SFM pendant 2 jours.

Le système HX/XO (Sigma, France) est ajouté dans une solution saline de Hank's, pH 7,4, et utilisé aux concentrations de 8 mU XO/ml et 80 µg d'hypoxanthine/ml.

Le système HX/XO est ajouté après lavage des cellules, laissé 150 min en contact avec les cellules puis stoppé par éviction des puits et rinçage des cellules.

La viabilité avec et sans produit est mesurée par un test colorimétrique MTT comme décrit à l'exemple 3 ci-dessus.

Le produit à tester, à savoir l'extrait $I_3$ de l'exemple 2, est administré à 1,25 µg/ml et 5 µg/ml dans du DMSO pendant 48 h avant le stress oxydatif de HX/XO et pendant le stress.

Les résultats obtenus sont répertoriés au tableau III ci-après.

TABLEAU III

| Produit | Viabilité cellulaire sans HX/XO | Viabilité cellulaire avec HX/XO | Significativité |
|---|---|---|---|
| Témoin | 100 % | 40 % | |
| Extrait $I_3$ de l'invention à 1,25 µg/ml | 100 % | 56 % | S* |
| Extrait $I_3$ de l'invention à 5 µg/ml | 100 % | 81 % | S* |

S* = significatif au seuil de 5 %

Il ressort clairement du tableau III que :

    a) le système HX/XO dans les conditions d'expérience tue 60 % des cellules, 40 % seulement restant en vie.

b) A 1,25 μg/ml l'extrait $I_3$ de l'invention protège déjà fortement les kératinocytes de l'effet toxique du système HX/XO, 56 % des cellules survivant à l'agression contre 40 % dans les cultures témoins, cette protection étant ainsi significative en démontrant ainsi la propriété antiradicalaire de l'extrait $I_3$ selon l'invention.

c) A 5 μg/ml, cet effet est d'autant plus important que 81 % des cellules survivent contre 40 % dans le contrôle.

Ainsi, on peut observer que les extraits de Bertholletia selon l'invention aboutissent à un pourcentage remarquable de cellules survivantes à l'agression et démontrent bien les propriétés antiradicalaires de ces extraits et leur intérêt pour le soin de la peau.

Ainsi, les extraits de Bertholletia excelsa peuvent avantageusement être utilisés, grâce à leur propriété de stimulation de la synthèse du collagène, comme agent actif, dans des compositions cosmétiques ou pharmaceutiques, notamment dermatologiques, telles que définies précédemment.

Diverses formulations de compositions cosmétiques sont données ci-après :

Exemple 6

Gel de massage raffermissant cutané

- Extrait d'écorce de Bertholletia excelsa $I_2$ de l'exemple 1        0,1 g
- Ethanol        5 g
- Glycérine        2g
- Propylèneglycol        2 g
- Carbopol 940®        1,25 g
- Excipient aqueux avec conservateur éventuellement parfumé        q.s.p. 100,00 g

Une partie de l'excipient aqueux est utilisée avec le Carbopol pour préparer séparément un gel, l'autre partie de l'excipient aqueux est utilisée pour être mélangée avec les autres composants et le gel est ajouté dans la solution obtenue afin d'obtenir une composition gélifiée formant le gel de massage.

Cette composition de gel de massage peut être utilisée trois fois par semaine pendant deux mois au niveau du buste.

Exemple 7

Lotion de soins du corps pour le raffermissement cutané

- Extrait d'écorce de Bertholletia excelsa $I_1$ de l'exemple 1        0,5 g
- Ethanol        1 g
- Acide hyaluronique        0,5 g
- Excipient aqueux contenant un conservateur éventuellement parfumé        q.s.p. 100,00 g

L'extrait est tout d'abord solubilisé dans l'agent solubilisant, puis est ajouté dans l'excipient aqueux auquel on ajoute l'acide hyaluronique.

La lotion obtenue peut être utilisée par cure de trois semaines sur les zones sensibles au relâchement, telles que le ventre, les cuisses.

Exemple 8

Emulsion anti-rides

- Extrait d'écorce de Bertholletia excelsa $I_2$ de l'exemple 1        0,025 g
- Excipient émulsionné parfumé        q.s.p. 100,00 g

Exemple 9

Composition cicatrisante

- Extrait d'écorce de Bertholletia excelsa $I_1$ de l'exemple 1        0,5 g
- Excipient émulsionné, type eau dans l'huile        q.s.p. 100,00 g

Exemple 10

Crème raffermissante et embellissante

- Extrait d'écorce de tronc de Bertholletia excelsa $I_2$ de l'exemple 1        0,1 g
- Asiaticoside        0,05 g
- Phosphate d'ascorbyle (sel de magnésium)        0,1 g
- Proline        0,05 g
- Excipients émulsionnés, avec conservateur,        q.s.p. 100,00 g

Cette crème, appliquée une ou deux fois par jour par cure de trois semaines, restaure les qualités de fermeté, d'élasticité ainsi que l'éclat d'une peau jeune.

Exemple 11

Crème anti-âge

- Extrait de péricarpe de noix de Bertholletia excelsa, $I_3$ de l'exemple 2        0,2g
- Ascorbate de phosphate de magnésium        1 g
- Acide hyaluronique        1 g
- Excipient émulsionné parfumé avec conservateur qsp        100 g

Cette crème, appliquée une ou deux fois par jour par cure de 3 semaines, localement au niveau du cou et du bas du visage, restaure les qualités de fermeté, l'élasticité ainsi que l'éclat d'une peau jeune.

Exemple 12

Crème de soin du visage anti-ride

- Extrait de péricarpe de noix de Bertholletia excelsa, $I_3$ de l'exemple 2        0,1 g
- Alpha tocophérol        0,05 g
- Vitamine C sous forme de sel phosphate de magnésium        0,1 g
- Madécassoside        0,1 g
- Palmitate de vitamine A        0,003g
- Excipient aqueux contenant un conservateur éventuellement parfumé        qsp 100,00 g

Cette crème, préparée de manière classique, appliquée une ou deux par jours par cure de 3 semaines sur les rides du visage, permet de restaurer les qualités de fermeté, d'élasticité ainsi que l'éclat d'une peau jeune en éliminant ou atténuant les rides.

**Revendications**

1. Utilisation d'un extrait de Bertholletia, comme agent cosmétique, notamment à activité antiradicalaire, en particulier pour le soin de la peau.

2. Utilisation d'un extrait de Bertholletia, en particulier de Bertholletia excelsa, pour la préparation d'une composition cosmétique ou pharmaceutique, notamment dermatologique, stimulant notamment la synthèse du collagène, en particulier celle du collagène I ou du collagène IV, ou ayant une activité antiradicalaire, et destinée notamment à améliorer la cicatrisation cutanée, ainsi que les propriétés biomécaniques et l'aspect de la surface de la peau, à obtenir un raffermissement cutané, à renforcer la structure et les propriétés de la jonction dermo-épidermique, à lutter contre les effets du vieillissement cutané, ou à traiter les diverses pathologies accompagnées d'une déficience en collagène.

3. Utilisation selon la revendication 1 ou 2, caractérisée en ce que la composition précitée est une composition à effet anti-vieillissement cutané, en particulier anti-rides, destinée à prévenir l'apparition des rides ou en atténuer la profondeur.

4. Utilisation selon l'une des revendications 1 à 3, caractérisée en ce que l'extrait précité est choisi parmi le groupe consistant d'un extrait d'écorce ou d'un extrait de noix, en particulier un extrait d'écorce de tronc ou un extrait de péricarpe de noix, de la plante Bertholletia, de préférence la plante Bertholletia excelsa.

5. Utilisation selon l'une des revendications 1 à 4, caractérisée en ce que l'extrait de Bertholletia précité est obtenu par extraction par un solvant polaire, avantageusement choisi parmi le groupe constitué par : l'eau, les alcools comportant de préférence de 1 à 4 atomes de carbone, tels que le méthanol ou l'éthanol, les solvants chlorés comportant de préférence de 1 à 2 atomes de carbone, tels que le chloroforme ou le dichlorométhane, les esters organiques comportant de préférence de 3 à 6 atomes de carbone, et d'un solvant mixte à base d'un mélange quelconque des solvants précités.

6. Utilisation selon la revendication 5, caractérisée en ce que le solvant polaire est choisi parmi le groupe constitué par l'eau, le méthanol, l'éthanol et un solvant mixte à base d'un mélange quelconque des solvants précités.

7. Utilisation selon l'une des revendications 1 à 6, caractérisée en ce que l'extrait de Bertholletia précité est enrichi en saponines.

8. Composition cosmétique, caractérisée en ce qu'elle comprend, à titre d'ingrédient actif, une quantité cosmétiquement efficace d'un extrait de Bertholletia, en particulier de Bertholletia excelsa, dispersé dans un excipient cosmétiquement acceptable.

9. Composition pharmaceutique, notamment dermatologique, stimulant la synthèse du collagène, en particulier celle du collagène I ou du collagène IV, ou à activité antiradicalaire, caractérisée en ce qu'elle comprend, à titre d'ingrédient actif, une quantité pharmaceutiquement efficace d'un extrait de Bertholletia, en particulier de Bertholletia excelsa, dispersé dans un excipient pharmaceutiquement acceptable.

10. Composition selon l'une des revendications 8 à 9, caractérisée en ce que la concentration en extrait de Bertholletia, en particulier de Bertholletia excelsa, est comprise entre 0,0001 % et 1 % en poids, de préférence entre 0,01 % et 0,25 % en poids, par rapport au poids total de ladite composition.

11. Composition selon l'une des revendications 8 à 10, caractérisée en ce que l'extrait précité est choisi parmi le groupe consistant d'un extrait d'écorce ou d'un extrait de noix, en particulier un extrait d'écorce de tronc ou un extrait de péricarpe de noix, de la plante Bertholletia, de préférence la plante Bertholletia excelsa.

12. Composition selon l'une des revendications 8 à 11, caractérisée en ce que l'extrait de Bertholletia, en particulier de Bertholletia excelsa précité, est obtenu par extraction par un solvant polaire, avantageusement choisi parmi le groupe constitué par : l'eau, les alcools comportant de préférence de 1 à 4 atomes de carbone, tels que le méthanol ou l'éthanol, les solvants chlorés comportant de préférence de 1 à 2 atomes de carbone, tels que le chloroforme ou le dichlorométhane, les esters organiques comportant de préférence de 3 à 6 atomes de carbone, et d'un solvant mixte à base d'un mélange quelconque des solvants précités.

13. Composition selon la revendication 12, caractérisée en ce que solvant polaire est choisi parmi le groupe constitué par l'eau, le méthanol, l'éthanol et un solvant mixte à base d'un mélange quelconque des solvants précités.

14. Composition selon l'une des revendications 8 à 13, caractérisée en ce que l'extrait de Bertholletia précité est enrichi en saponines.

15. Composition selon l'une des revendications 8 à 14, caractérisée en ce qu'elle contient en outre une substance active choisie parmi le groupe constitué par l'acide ascorbique, l'acide madécassique, l'acide asiatique, le madécassoside, l'asiaticoside, l'alpha-1-protéinase inhibiteur, les inhibiteurs de collagénase tels que l'acide rétinoïque, les inhibiteurs d'élastase, la lysine, la proline, le 2-oxoglutarate et le ginsénoside $R_0$.

16. Procédé de traitement cosmétique, caractérisé en ce qu'il comprend l'application topique sur une zone externe du corps humain d'une quantité cosmétiquement efficace d'un extrait de Bertholletia, en particulier Bertholletia excelsa, en particulier dispersée dans un excipient cosmétiquement acceptable.

17. Procédé selon la revendication 16, caractérisé en ce que l'extrait de Bertholletia, en particulier de Bertholletia excelsa, est présent dans une composition comprenant une concentration en extrait de Bertholletia, en particulier de Bertholletia excelsa, comprise entre 0,0001 % et 1 %, de préférence entre 0,01 % et 0,25 %, en poids par rapport au poids total de la composition finale.

18. Procédé selon la revendication 16 ou 17, caractérisé en ce qu'il s'agit d'un traitement cosmétique pour stimuler la synthèse du collagène, en particulier celle du collagène I ou du collagène IV, ou à activité antiradicalaire, pour améliorer la cicatrisation cutanée, pour lutter contre les effets du vieillissement cutané, pour prévenir l'apparition des rides ou en atténuer la profondeur, pour obtenir un raffermissement cutané ou pour renforcer la structure et les propriétés de la jonction dermo-épidermique.


**Patentansprüche**

1. Verwendung eines Bertholletia-Extrakts als kosmetisches Mittel, insbesondere mit Radikalschutz-Aktivität, insbesondere für die Hautpflege.

2. Verwendung eines Extrakts von Bertholletia, insbesondere von Bertholletia excelsa, für die Herstellung einer kosmetischen oder pharmazeutischen, insbesondere dermatologischen Zusammensetzung, die insbesondere die Synthese von Kollagen, insbesondere jene von Kollagen I oder Kollagen IV stimuliert oder die eine Radikalschutz-Aktivität aufweist und die insbesondere dafür bestimmt ist, die Narbenbildung der Haut wie auch die biomechanischen Eigenschaften und das Aussehen der Hautoberfläche zu verbessern, eine Kräftigung der Haut zu erzielen, die Struktur und die Eigenschaften der Verbindungsstelle zwischen Dermis und Epidermis zu verstärken, die Auswirkungen der Hautalterung zu bekämpfen oder verschiedene Erkrankungen, die von einem Kollagenmangel begleitet werden, zu behandeln.

3. Verwendung nach Anspruch 1 oder 2, dadurch gekennzeichnet, daß die Zusammensetzung eine Zusammensetzung mit einer Anti-Hautalterungswirkung, insbesondere Antifalten-Wirkung ist, die dazu bestimmt ist, das Auftreten der Falten zu verhindern oder deren Tiefe zu mildern.

4. Verwendung nach einem der Ansprüche 1 bis 3, dadurch gekennzeichnet, daß der Extrakt ausgewählt wird aus der Gruppe, bestehend aus einem Rindenextrakt oder einem Nussextrakt, insbesondere einem Extrakt von Stammrinde oder einem Extrakt des Samengehäuses der Nuss von der Pflanze Bertholletia, vorzugsweise der Pflanze Bertholletia excelsa.

5. Verwendung nach einem der Ansprüche 1 bis 4, dadurch gekennzeichnet, daß der Bertholletia-Extrakt erhalten wird durch Extraktion durch ein polares Lösemittel, vorteilhafterweise ausgewählt aus der Gruppe, gebildet aus: Wasser, den Alkoholen, die vorzugsweise 1 bis 4 Kohlenstoffatome umfassen, wie beispielsweise Methanol oder Ethanol, den chlorhaltigen Lösemitteln, die vorzugsweise 1 bis 2 Kohlenstoffatome umfassen, wie beispielsweise Chloroform oder Dichlormethan, den organischen Estern, die vorzugsweise 3 bis 6 Kohlenstoffatome umfassen, und einem gemischten Lösemittel auf Basis einer beliebigen Mischung der vorstehend genannten Lösemittel.

6. Verwendung nach Anspruch 5, dadurch gekennzeichnet, daß das polare Lösemittel ausgewählt wird aus der Gruppe, gebildet aus Wasser, Methanol, Ethanol und einem gemischten Lösemittel auf Basis einer beliebigen Mischung der vorstehend genannten Lösemittel.

7. Verwendung nach einem der Ansprüche 1 bis 6, dadurch gekennzeichnet, daß der Bertholletia-Extrakt an Saponinen angereichert ist.

8. Kosmetische Zusammensetzung, dadurch gekennzeichnet, daß sie als Wirkstoff eine kosmetisch wirksame Menge eines Extrakts von Bertholletia, insbesondere von Bertholletia excelsa, dispergiert in einer kosmetisch annehmbaren Trägersubstanz, umfaßt.

9. Pharmazeutische, insbesondere dermatologische Zusammensetzung, die die Synthese von Kollagen, insbesondere jene von Kollagen I oder Kollagen IV stimuliert, oder mit einer Radikalschutz-Aktivität, dadurch gekennzeichnet, daß sie als Wirkstoff eine pharmazeutisch wirksame Menge eines Extrakts von Bertholletia, insbesondere von Bertholletia excelsa, dispergiert in einer pharmazeutisch annehmbaren Trägersubstanz, umfaßt.

10. Zusammensetzung nach einem der Ansprüche 8 bis 9, dadurch gekennzeichnet, daß die Konzentration an Extrakt von Bertholletia, insbesondere von Bertholletia excelsa zwischen 0,0001 Gew.-% und 1 Gew.-%, vorzugsweise zwischen 0,01 Gew.-% und 0,25 Gew.-% bezogen auf das Gesamtgesicht der Zusammensetzung ist.

11. Zusammensetzung nach einem der Ansprüche 8 bis 10, dadurch gekennzeichnet, daß der Extrakt ausgewählt ist aus der Gruppe, bestehend aus einem Rindenextrakt oder einem Nussextrakt, insbesondere einem Extrakt von Stammrinde oder einem Extrakt des Samengehäuses der Nuss von der Pflanze Bertholletia, vorzugsweise der Pflanze Bertholletia excelsa.

12. Zusammensetzung nach einem der Ansprüche 8 bis 11, dadurch gekennzeichnet, daß der Extrakt von Bertholletia, insbesondere von Bertholletia excelsa erhalten wird durch Extraktion durch ein polares Lösemittel, vorteilhafterweise ausgewählt aus der Gruppe, gebildet aus: Wasser, den Alkoholen, die vorzugsweise 1 bis 4 Kohlenstoffatome umfassen, wie beispielsweise Methanol oder Ethanol, den chlorhaltigen Lösemitteln, die vorzugsweise 1 bis 2 Kohlenstoffatome umfassen, wie beispielsweise Chloroform oder Dichlormethan, den organischen Estern, die vorzugsweise 3 bis 6 Kohlenstoffatome umfassen, und einem gemischten Lösemittel auf Basis einer beliebigen Mischung der vorstehend genannten Lösemittel.

13. Zusammensetzung nach Anspruch 12, dadurch gekennzeichnet, daß das polare Lösemittel ausgewählt ist aus der Gruppe, gebildet aus Wasser, Methanol, Ethanol und einem gemischten Lösemittel auf Basis einer beliebigen Mischung der vorstehend genannten Lösemittel.

14. Zusammensetzung nach einem der Ansprüche 8 bis 13, dadurch gekennzeichnet, daß der Bertholletia-Extrakt an Saponinen angereichert ist.

15. Zusammensetzung nach einem der Ansprüche 8 bis 14, dadurch gekennzeichnet, daß sie darüber hinaus einen Wirkstoff, ausgewählt aus der Gruppe, gebildet aus Ascorbinsäure, Madecassinsäure, Asiatsäure, Madecassosid, Asiaticosid, $\alpha$-1-Proteinase-Inhibitor, den Kollagenase-Inhibitoren, wie Retinsäure, den Elastase-Inhibitoren, Lysin, Prolin, 2-Oxoglutarat und Ginsenosid $R_0$, enthält.

16. Kosmetisches Behandlungsverfahren, dadurch gekennzeichnet, daß es das topische Auftragen einer kosmetisch wirksamen Menge eines Extrakts von Bertholletia, insbesondere von Bertholletia excelsa, insbesondere in einer kosmetisch annehmbaren Trägersubstanz dispergiert, auf einen äußerlichen Bereich des menschlichen Körpers umfaßt.

17. Verfahren nach Anspruch 16, dadurch gekennzeichnet, daß der Extrakt von Bertholletia, insbesondere von Bertholletia excelsa in einer Zusammensetzung vorliegt, die eine Konzentration an Extrakt von Bertholletia, insbesondere von Bertholletia excelsa, zwischen 0,0001 Gew.-% und 1 Gew.-%, vorzugsweise zwischen 0,01 Gew.-% und 0,25 Gew.-% bezogen auf das Gesamtgewicht der endgültigen Zusammensetzung umfaßt.

18. Verfahren nach Anspruch 16 oder 17, dadurch gekennzeichnet, daß es sich um eine kosmetische Behandlung zur Stimulierung der Kollagensynthese, insbesondere von jener von Kollagen I oder Kollagen IV, oder mit Radikalschutz-Aktivität zur Verbesserung der Narbenbildung der Haut, zur Bekämpfung der Auswirkungen der Hautalterung, zur Verhinderung des Auftretens von Falten oder zum Lindern von deren Tiefe, zur Erzielung einer Haut-

**EP 0 809 484 B1**

kräftigung oder zur Verstärkung der Struktur und der Eigenschaften der Verbindungsstelle zwischen Dermis und Epidermis handelt.

**Claims**

1. Use of an extract of Bertholletia as a cosmetic agent, especially with anti-free radical activity, in particular for skin care.

2. Use of an extract of Bertholletia, particularly Bertholletia excelsa, for the preparation of a cosmetic or pharmaceutical composition, especially a dermatological composition, which stimulates especially the synthesis of collagen, particularly collagen I or collagen IV, or has anti-free radical activity, and which is intended especially for improving skin healing as well as the biomechanical properties and surface appearance of the skin, obtaining firmer skin, strengthening the structure and properties of the epidermal-dermal junction, combating the effects of skin ageing or treating the diverse pathological conditions accompanied by a collagen deficiency.

3. Use according to claim 1 or 2, characterised in that the above-mentioned composition is a composition which is effective against skin ageing, particularly against wrinkles, and which is intended for preventing the appearance of wrinkles or reducing their depth.

4. Use according to one of claims 1 to 3, characterised in that the above-mentioned extract is selected from the group comprising an extract of the bark or an extract of the nuts, particularly an extract of the bark of the trunk or an extract of the pericarp of the nuts, of the Bertholletia plant, preferably the Bertholletia excelsa plant.

5. Use according to one of claims 1 to 4, characterised in that the above-mentioned extract of Bertholletia is obtained by extraction with a polar solvent advantageously selected from the group comprising water, alcohols preferably containing from 1 to 4 carbon atoms, such as methanol or ethanol, chlorinated solvents preferably containing 1 or 2 carbon atoms, such as chloroform or dichloromethane, organic esters preferably containing from 3 to 6 carbon atoms, and a mixed solvent based on any mixture of the above-mentioned solvents.

6. Use according to claim 5, characterised in that the polar solvent is selected from the group comprising water, methanol, ethanol and a mixed solvent based on any mixture of the above-mentioned solvents.

7. Use according to one of claims 1 to 6, characterised in that the above-mentioned extract of Bertholletia is enriched in saponins.

8. Cosmetic composition, characterised in that it comprises, as the active ingredient, a cosmetically effective amount of an extract of Bertholletia, particularly Bertholletia excelsa, dispersed in a cosmetically acceptable excipient.

9. Pharmaceutical composition, especially dermatological composition, which stimulates the synthesis of collagen, particularly collagen I or collagen IV, or which has anti-free radical activity, characterised in that it comprises, as the active ingredient, a pharmaceutically effective amount of an extract of Bertholletia, particularly Bertholletia excelsa, dispersed in a pharmaceutically acceptable excipient.

10. Composition according to one of claims 8 or 9, characterised in that the concentration of extract of Bertholletia, particularly Bertholletia excelsa, is between 0.0001% and 1% by weight, preferably between 0.01% and 0.25% by weight, based on the total weight of said composition.

11. Composition according to one of claims 8 to 10, characterised in that the above-mentioned extract is selected from the group comprising an extract of the bark or an extract of the nuts, particularly an extract of the bark of the trunk or an extract of the pericarp of the nuts, of the Bertholletia plant, preferably the Bertholletia excelsa plant.

12. Composition according to one of claims 8 to 11, characterised in that the above-mentioned extract of Bertholletia, particularly Bertholletia excelsa, is obtained by extraction with a polar solvent advantageously selected from the group comprising water, alcohols preferably containing from 1 to 4 carbon atoms, such as methanol or ethanol, chlorinated solvents preferably containing 1 or 2 carbon atoms, such as chloroform or dichloromethane, organic esters preferably containing from 3 to 6 carbon atoms, and a mixed solvent based on any mixture of the above-mentioned solvents.

**13.** Composition according to claim 12, characterised in that the polar solvent is selected from the group comprising water, methanol, ethanol and a mixed solvent based on any mixture of the above-mentioned solvents.

**14.** Composition according to one of claims 8 to 13, characterised in that the above-mentioned extract of Bertholletia is enriched in saponins.

**15.** Composition according to one of claims 8 to 14, characterised in that it also contains an active substance selected from the group comprising ascorbic acid, madecassic acid, asiatic acid, madecassoside, asiaticoside, alpha-1-proteinase inhibitor, collagenase inhibitors such as retinoic acid, elastase inhibitors, lysine, proline, 2-oxoglutarate and ginsenoside $R_0$.

**16.** Method of cosmetic treatment, characterised in that it comprises the topical application, to an external area of the human body, of a cosmetically effective amount of an extract of Bertholletia, especially Bertholletia excelsa, particularly dispersed in a cosmetically acceptable excipient.

**17.** Method according to claim 16, characterised in that the extract of Bertholletia, particularly Bertholletia excelsa, is present in a composition which comprises a concentration of extract of Bertholletia, particularly Bertholletia excelsa, of between 0.0001% and 1% by weight, preferably of between 0.01% and 0.25% by weight, based on the total weight of the final composition.

**18.** Method according to claim 16 or 17, characterised in that it involves a cosmetic treatment which stimulates the synthesis of collagen, particularly collagen I or collagen IV, or which has anti-free radical activity, for improving skin healing, combating the effects of skin ageing, preventing the appearance of wrinkles or reducing their depth, obtaining firmer skin or strengthening the structure and properties of the epidermal-dermal junction.